(19) Europäisches Patentamt
European Patent Office
Office européen des brevets

(11) Publication number: 0 355 842
A2

(12) # EUROPEAN PATENT APPLICATION

(21) Application number: 89115710.9

(22) Date of filing: 25.08.89

(51) Int. Cl.4: A61K 7/42

(30) Priority: 26.08.88 JP 213255/88
09.11.88 JP 284127/88
09.11.88 JP 284128/88
10.11.88 JP 284964/88

(43) Date of publication of application:
28.02.90 Bulletin 90/09

(84) Designated Contracting States:
DE FR GB IT

(71) Applicant: Sansho Seiyaku Co., Ltd.
26-7, Oike 2-chome
Ohnojo-shi Fukuoka-ken(JP)

(72) Inventor: Oyama, Yasuaki
15-16, Oike 2-chome
Onojo-shi Fukuoka-ken(JP)
Inventor: Okazaki, Kiyotaka
7-25-572, Kamiori 2-chome
Onojo-shi Fukuoka-ken(JP)

(74) Representative: Patentanwälte Deufel- Schön-Hertel- Lewald- Otto
Isartorplatz 6
D-8000 München 2(DE)

(54) **External preparation.**

(57) This invention provides an external preparation effectively preventing skin pigmentation caused by melanin production, i.e. spots or freckle, and having also superior actions on fair-skin making up, the external preparation containing unsaturated straight-chain fatty acids or unsaturated straight-chain aliphatic alcohols or the derivatives thereof as effective ingredients.

EP 0 355 842 A2

# EXTERNAL PREPARATION

## BACKGROUND OF THE INVENTION

### Field of the Invention:

This invention relates to an external preparation which is able to effectively prevent skin pigmentation caused by melanin production, i.e. spots or freckle, and which also has superior actions on fair-skin making up, the external preparation containing unsaturated straight-chain fatty acids or unsaturated straight-chain aliphatic alcohols or their derivatives as effective ingredients.

### Prior Art:

There have been conventionally known kojic acid and kojic acid derivatives as inhibitors on melanin production.

Various kinds of fair-skin making cosmetics containing these substances as effective ingredients have been disclosed (e.g. Patent Publication Sho. 56-18569, Published unexamined Patent Publication Sho. 56-7710, Published unexamined Patent Publication Sho. 56-7776, Published unexamined Patent Publication Sho. 56-79616). There have also been known flavonol compounds. Some cosmetics and dermal preparations containing quercetin as an effective ingredient are described in Published unexamined Patent Publication Sho. 55-92305 and Published unexamined Patent Publication Sho. 57- 14517). Those Containing quercetin fatty acid ester as effective ingredients are described in Patent Publication Sho. 58-34477 and those containing myrisetin, fisetin, gutiscetin, rhamnetin and the like as effective ingredients are described in Published unexamined Patent Publication Sho. 55-111410 and Published unexamined Patent Publication Sho. 55-35506. There have been further disclosed those containing 3-hydroxychromone compounds as effective ingredients in Published unexamined Patent Publications Sho. 55-111410 and Sho. 55-143908.

## SUMMARY OF THE INVENTION

The inventors have now investigated, safe substances possessing inhibiting activity on melanin production and examined their efficiency as external preparations. Thus we found that unsaturated straight-chain fatty acids, unsaturated straight-chain aliphatic alcohols and their derivatives have superior inhibiting activity on melanin production. An object of the present invention is to provide external preparations containing such substances as active ingredients.

After the extensive investigation for substances inhibiting melanin production, the inventors found that unsaturated straight-chain fatty acids, unsaturated straight-chain aliphatic alcohols and their derivatives possessing carbon atoms of 4 to 20 and unsaturated bonds of 1 to 4, have strong inhibiting activity on melanin production. Thus the inventors achieved the invention.

The invention provides external preparations containing, as effective ingredients, unsaturated straight-chain fatty acids and unsaturated straight-chain aliphatic alcohols possessing carbon atoms of 4 to 20 and unsaturated bonds of 1 to 4 at appropriate positions other than the position of carboxyl group or hydroxyl group of alkyl chain and the terminal position opposite thereto.

The unsaturated straight-chain fatty acids as effective ingredients according to the invention have carbon atoms of 4 to 20 and unsaturated bonds of 1 to 4 at appropriate positions other than the position of carboxyl group of alkyl chain and the terminal position opposite thereto; they include, for example, 2-butenoic acid, 6-octenoic acid, 6-decenoic acid, 7-undecylenic acid, 9-dodecenoic acid, 6,9-dodecadienoic acid, 7,10-pentadecadienoic acid, 10-heptadecenoic acid, 9-hexadecenoic acid, 6,9-hexadecadienoic acid, 6,9,12-hexadecatrienoic acid, 6-octadecenoic acid, 11-octadecenoic acid, 11,14-eicosadienoic acid, 8,11,14-eicosatrienoic acid, 11,14,17-eicosatrienoic acid, 5,8,11,14-eicosatetraenoic acid, 5,8,11,14,17-eicosapentaenoic acid and the like.

The derivatives of these unsaturated straight-chain fatty acids according to the invention include the following compounds;

(1) monoglycerides shown in Formula (I) or (II)

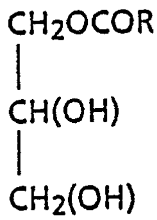

(I)

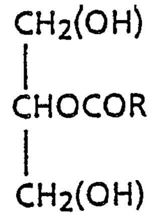

(II)

(wherein R represent aliphatic group of the above unsaturated straight-chain fatty acids having 4 to 20 carbon atoms and unsaturated bonds of 1 to 4),

(2) diglycerides shown in formula (III) or (IV)

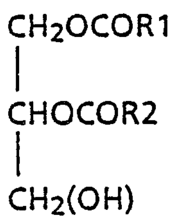

(III)

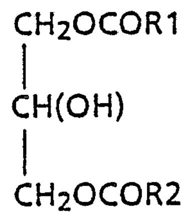

(IV)

(wherein R1 and R2 are chain organic groups,preferably aliphatic groups of saturated and unsaturated fatty acids having 10 to 26 carbon atoms and at least one of these groups represents aliphatic group of the above unsaturated straight-chain fatty acids having 4 to 20 carbon atoms and unsaturated bonds of 1 to 4),

(3) triglycerides shown in formula (V)

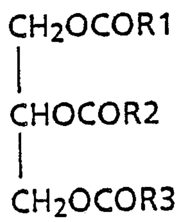

(V)

(wherein R1, R2 and R3 are chain organic groups, preferably aliphatic groups of saturated and unsaturated fatty acids having 10 to 26 carbon atoms and at least one of these groups represents aliphatic group of the above unsaturated straight-chain fatty acids having 4 to 20 carbon atoms and unsaturated bonds of 1 to 4),

(4) metal salts shown in formula (VI)

(RCOO)pM (VI)

(wherein R represents aliphatic group of the above unsaturated straight-chain fatty acids having 4 to 20 carbon gatoms and unsaturated bonds of 1 to 4 and M represents a metal atom and p represents M's valence),

(5) esters shown in formula (VII)

$RCOOR'$ (VII)

(R represents aliphatic group of the above unsaturated straight-chain fatty acids; $R'$ represents a residual group of polyalcohol, polyoxyethylene, sorbitan or sugar with one or two or more valence),

(6) phospholipid shown in formula (VIII)

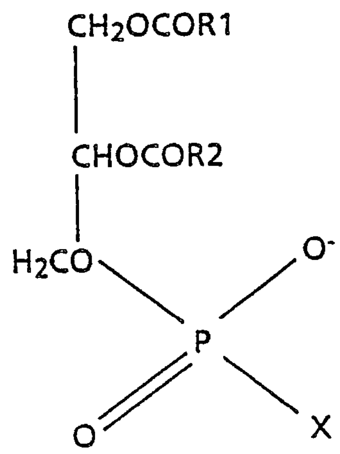

(VIII)

(wherein R1 and R2 are chain organic groups, preferably aliphatic groups of saturated and unsaturated fatty acids having 10 to 26 carbon atoms and at least one of these groups represents aliphatic group of the above unsaturated straight-chain fatty acids having 4 to 20 carbon atoms and unsaturated bonds of 1 to 4), X represents a residual group of choline, ethanolamine, resine, glycerol, phospholipid or inositol), (7) phosphatidic acid shown in formula (IX)

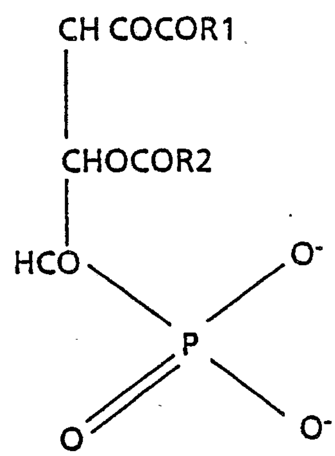

(IX)

(wherein R1 and R2 are chain organic groups, preferably aliphatic groups of saturated and unsaturated fatty acids having 10 to 26 carbon atoms and at least one of these groups represents aliphatic group of the above unsaturated straight-chain fatty acids having 4 to 20 carbon atoms and unsaturated bonds of 1 to 4),

(8) sterolesters shown in formula (X)

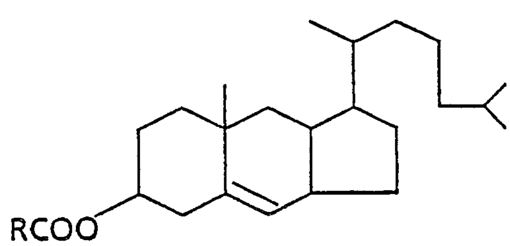

(X)

(R represents aliphatic group of the unsaturated straight-chain fatty acids having 4 to 20 carbon atoms and unsaturated bonds of 1 to 4),

(9) sphingolipid shown in formula (XI)

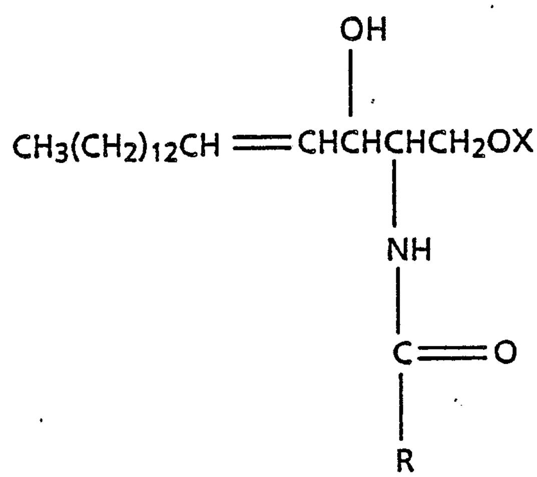

(XI)

(R represents aliphatic group of the unsaturated straight-chain fatty acids having 4 to 20 carbon atoms and unsaturated bonds of 1 to 4; X represents a residual group of sugar, phosphoric acid or amine salts),

(10) primary amides shown in formula (XII)

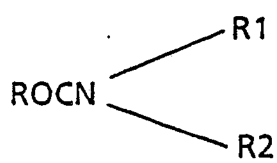

(XII)

(R represents aliphatic group of the unsaturated straight-chain fatty acids having 4 to 20 carbon atoms and unsaturated bonds of 1 to 4; R′ and R″ represent hydrogen atom or organic groups),

(11) secondary amides shown in formula (XIII)

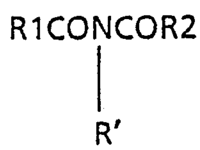

(XIII)

(wherein R1 and R2 are chain groups, preferably aliphatic groups of saturated and unsaturated fatty acids having 10 to 26 carbon atoms and at least one of these groups represents aliphatic group of the above unsaturated straight- chain fatty acids having 4 to 20 carbon atoms and unsaturated bonds of 1 to 4; R′ represents hydrogen atom or organic groups),

(12) tertiary amides shown in formula (XIV)

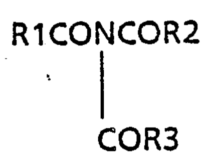

(XIV)

(wherein R1, R2 and R3 are chain organic groups, preferably aliphatic groups of saturated and unsaturated fatty acids having 10 to 26 carbon atoms and at least one of these groups represetns aliphatic group of the above unsaturated straight-chain fatty acids having 4 to 20 carbon atoms and unsaturated bonds of 1 to 4); R′ represents hydrogen atom or organic groups),

(13) dibasic acids and their salts shown in formula (XV)

HOOCRCOOH (XV)

(R represents aliphatic group of the above unsaturated straight-chain fatty acids having 4 to 20 carbon atoms and unsaturated bonds of 1 to 4).

These derivatives include monoglycerides such as 1-mono-6-octenoin, 1-mono-9-dodecenoin, 1-monomyristolein, 1-mono-10-pentadecenoin, 1-monopalmitolein, 1-mono-10-heptadecenoin, 2-mono-3-hexenoin, 2-mono-7-undecenoin, 2-monopentadecenoin, 2-monopalmitolein, 2-mono-10-heptadecenoin, 1,2-di-9-dodecenoin, 1-mono-6-octadecenoin, 1-mono-11-octadecenoin, 1-mono-11,14-eicosadienoin, 1-mono-11,14,17-eicosatrienoin, 1-mono-8,11,14,17-eicosatetraenoin, 1-mono-5,8,11,14,17-eicosapentaenoin, 2-mono-6-octadecenoin, 2-mono-11-octadecenoin, 2-monoeicosa-11,14-dienoin, 2-monoeicosa-8,11,14-eicosatrienoin, 2-mono-11,14,17-eicosatrienoin, 2-mono-8,11,14 eicosatetraenoin, 2-mono-5,8,11,14,17-eicosapentaenoin and the like; diglycerides such as 1,2-dimyristolein, 1,2-di-10-pentadecenoin, 1,2-dipalmitolein, 1,3-dimyristolein, 1,3-di-10-pentadecenoin, 1,3-dipalmitolein, 1,3-di-10-heptadecenoin, 1,2-di-6-octadecenoin, 1,2-di-11-octadecenoin, 1,2-di-11,14-eicosadienoin, 1,2-di-8,11,14-eicosatrienoin, 1,2-di-11,14,17-eicosatrienoin, 1,2-di-8,11,14,17-eicosatetraenoin, 1,2-di-5,8,11,14,17-eicosapentaenoin and the like; triglycerides such as trimyristolein, tri-10-pentadecenoin, tripalmitolein, tri-10-heptadecenoin, tri-6-octadecenoin, tri-11-octadecenoin, tri-11,14-eicosadienoin, tri-8,11,14-eicosatrienoin, tri-11,14,17-eicosatrienoin, tri-8,11,14,17-eicosatetraenoin, tri-5,8,11,14,17-eicosapentaenoin; esters such as 9-myristoleic acid methyl, 10-pentadecenoic acid methyl, palmitoleic acid methyl, 10-heptadecenoic acid methyl, 6-octadecenoic acid methyl, 11-octadecenoic acid methyl, 11,14-eicosadienoic acid methyl, 8,11,14-eicosatrienoic acid methyl, 11,14,17-eicosatrienoic acid methyl, 5,8,11,14-eicosatetraenoic acid methyl, 5,8,11,14,17-eicosapentaenoic acid methyl and the like; phosphatidic acids such as dipalmitoleylphosphatidic acid disodium, di-10-pentadecenylphosphatidic acid disodium, di-6-octadecenoylphosphatidic acid disodium salt, di-5,8,11,14,17-eicosapentanoylphosphatidic acid disodium salt and the like; sphingolipids such as N-palmitoleyl-D-sphingomielin, N-10-pentadecenyl-D-sphingomielin, N-6-octadecenoyl-D-sphingomielin, N-5,8,11,14-eicosatetraenyl-D-sphingomielin, N-5,8,11,14,17-eicosapentaenyl-D-sphingomielin and the like; phospholipids such as di-6-octadecenoyl-L-α-lecithin, di-11-octadecenoyl-L-α-lecithin, di-11,14-eicosadienoyl-L-α-lecithin, di-eicosenoyl-L-α-lecithin, di-8,11,14-eicosatrienoyl-L-α-lecithin, di-11,14,17-eicosatrienoyl-L-α-lecithin, di-5,8,11,14-eicosatetraenoyl-L-α-lecithin, di-5,8,11,14,17-eicosapentaenoyl-L-α-lecithin; amides such as palmitoleic acid amide, dipalmitoleic acid amide, tripalmitoleic acid amide, 6-octadecenoic acid amide, 11-octadecenoic acid amide, 11,14-eicosadienoic acid amide, 8,11,14-eicosatrienoic acid amide, 11,14,17-eicosatrienoic acid amide, 5,8,11,14-eicosatetraenoic acid amide, 5,8,11,14,17-eicosapentaenoic acid amide, di-6-octadecenoic acid amide, di-11-octadecenoic acid amide, di-11,14-eicosadienoic acid amide, di-11,14,17-eicosatrienoic acid amide, di-8,11,14-eicosatrienoic acid amide, di-5,8,11,14-eicosatetraenoic acid amide, di-5,8,11,14,17-eicosapentaenoic acid amide, tri-6-octadecenoic acid amide, tri-11-octadecenoic acid amide, tri-11,14-eicosadienoic acid amide, tri-8,11,14-eicosatrienoic acid amide, tri-11,14,17-eicosatrienoic acid amide, tri-5,8,11,14-eicosatetraenoic acid amide, tri-5,8,11,14,17-eicosapentaenoic acid amide; dicarboxylic acids such as 9-hexadecene dicarboxylic acid, 6-octadecene dicarboxylic acid, 11-octadecene dicarboxylic acid; sterolesters such as cholesteryl palmitoleate, cholesteryl myristoleate, cholesteryl 6-octadecenoate, cholesteryl 5,8,11,14-eicosatetraenoate, cholesteryl 5,8,11,14,17-eicosapentaenoate.

Unsaturated straight-chain aliphatic alcohols as effective ingredients in accordance with the invention have carbon atoms of 4 to 20 and unsaturated bonds of 1 to 4 at appropriate positions other than the position of hydroxyl group of alkyl chain and the terminal position opposite thereto; they include, for example, 3-hexenol, 6-decenol, 6-tetradecenol, 10-pentadecenol, 9-hexadecenol, 10-heptadecenol, 3,6-octadienol, 6,9-undecadienol, 6,9-tetradecadienol, 7,10-pentadecadienol, 6,9-hexadecadienol, 6,9,12-hexadecatrienol, 7,10,13-heptadecatrienol, 6-octadecenol, 11-octadecenol, 11,14-eicosadienol, 8,11,14-eicosatrienol, 11,14,17-eicosatrienol, 5,8,11,14-eicosatetraenol, 5,8,11,14-eicosapentaenol and the like.

The derivatives of the unsaturated straight-chain aliphatic alcohols as effective ingredients in accordance with the invention include esters, ethers, alkylmethane sulfonic acid ester, phosphonic acid of the above unsaturated straight-chain aliphatic alcohols and the like.

Ester compounds include acetyl esters of the above alcohols such as 6-octadecenyl acetyl ester, 11-octadecenyl acetyl ester, 11, 14-eicosadienyl acetyl ester, 11,14,17-eicosatrienyl acetyl ester, 5,8,11,14-eicosatrienyl acetyl ester, 5,8,11,14,17-eicosapentaenyl acetyl ester and the like.

Ether compounds include myristoleyl acetate, 10-pentadecenyl acetate, palmitolenoyl acetate, 10-heptadecenyl acetate, myristolenoyl methyl ether, 10-pentadecenyl ethyl ether, 9-hexadecenyl methyl ether, 10-heptadecenyl ethyl ether and the like.

Alkylmethane sulfonic acid esters include such as myristoleylmethane sulfonate, 10-pentadecenylmethane sulfonate, 10-heptadecenylmethane sulfonate and the like.

Phosphonic acids include myristoleylphosphonic acid , 10-pentadecenylsulfonic acid, palmitoleylphosphonic acid, 10-heptadecenylphosphonic acid.

The term external preparation according to the invention is used to include quasi drugs for external use such as emulsions, lotions and liniments as well as cosmetics, e.g. cream, lotion and pack.

The external preparation for cosmetics according to the invention contains effective ingredients of acids, alcohols or the derivatives thereof at a ratio of 0.001 - 5%, preferably 0.001 - 10%, by weight; in the case of quasi drugs, the ratio is 0.01 - 10%, preferably 0.01 - 2%, by weight.

The external preparation of the present invention may be prepared with base and coagents, conventionally used in the preparation of cosmetics and quasi drugs for external application by the rule of the art.

Furthermore, UV absorbing agents such as benzophenones, silicic acid compounds, salicylic acid compounds, benzoic acid compounds, urocanic acid ethyl, benzotriazole and others derived from plants may be preferably added, and also antioxidants such as vitamin E, BHA, BHT, erythorbic acid, filic acid, nordehydroguaiaretic acid, propyl gallate and the like may be added.

Examples of the invention are described and explained in more detail in the following.

| Example 1. Cream | |
|---|---|
| 9-Tetradecenoic acid | 0.05 % |
| Polyoxyethylene stearyl ether | 2.0 % |
| Polyoxyethylene cetyl ether | 2.0 % |
| Bee wax | 6.0 % |
| Cetanol | 6.0 % |
| Isopropyl palmitate | 10.0 % |
| Liquid paraffin | 30.0 % |
| Polyethylene glycol monostearate | 1.0 % |
| Paraoxybenzoic acid methyl | 0.2 % |
| Distilled water | ad. 100 |

| Example 2. Cream | |
|---|---|
| 6-Octadecenoic acid | 0.5 % |
| Polyoxyethylene stearyl ether | 2.0 % |
| Polyoxyethylene cetyl ether | 2.0 % |
| Bee wax | 6.0 % |
| Cetanol | 6.0 % |
| Isopropyl palmitate | 10.0 % |
| Liquid paraffin | 30.0 % |
| Polyethylene glycol monostearate | 1.0 % |
| Paraoxybenzoic acid methyl | 0.2 % |
| Distilled water | ad. 100 |

| Example 3. Cream | |
|---|---|
| 10-Pentadecenoic acid | 0.5 % |
| Polyoxyethylene stearyl ether | 2.0 % |
| Polyoxyethylene cetyl ether | 2.0 % |
| Bee wax | 6.0 % |
| Cetanol | 6.0 % |
| Isopropyl palmitate | 10.0 % |
| Liquid paraffin | 30.0 % |
| Polyethylene glycol monostearate | 1.0 % |
| Paraoxybenzoic acid methyl | 0.2 % |
| Distilled water | ad. 100 |

| Example 4. Cream | |
|---|---|
| 9-Hexadecenol | 0.05% |
| Polyoxyethylene stearyl ether | 2.0 % |
| Polyoxyethylene cetyl ether | 2.0 % |
| Bee wax | 6.0 % |
| Cetanol | 6.0 % |
| Isopropyl palmitate | 10.0 % |
| Liquid paraffin | 30.0 % |
| Polyethylene glycol monostearate | 1.0 % |
| Paraoxybenzoic acid methyl | 0.2 % |
| Distilled water | ad. 100 |

| Example 5. Emulsion | |
|---|---|
| 7,10-Pentadecadienoic acid | 0.1 % |
| Self-emulsifiable glycerol monostearate | 0.5 % |
| Polyoxyethylene cetyl ether | 2.0 % |
| MC stearic acid | 2.0 % |
| Cetanol | 1.5 % |
| Isopropyl myristate | 10.0 % |
| Paraoxybenzoic acid methyl | 0.2 % |
| Perfume | trace |
| Distilled water | ad. 100 |

| Example 6. Emulsion | |
|---|---|
| 11,14-Eicosadienoic acid | 0.1 % |
| Self-emulsifiable glycerol monostearate | 0.5 % |
| Polyoxyethylene cetyl ether | 2.0 % |
| MC stearic acid | 2.0 % |
| Cetanol | 1.5 % |
| Isopropyl myristate | 10.0 % |
| Paraoxybenzoic acid methyl | 0.2 % |
| Perfume | trace |
| Distilled water | ad. 100 |

| Example 7. Emulsion | |
|---|---|
| 7,10-Pentadecadienol | 0.1 % |
| Self-emulsifiable glycerol monostearate | 0.5 % |
| Polyoxyethylene cetyl ether | 2.0 % |
| MC stearic acid | 2.0 % |
| Cetanol | 1.5 % |
| Isopropyl myristate | 10.0 % |
| Paraoxybenzoic acid methyl | 0.2 % |
| Perfume | trace |
| Distilled water | ad. 100 |

| Example 8. Emulsion | |
|---|---|
| 9-Hexadecenoic acid | 0.1 % |
| Self-emulsifiable glycerol monostearate | 0.5 % |
| Polyoxyethylene cetyl ether | 2.0 % |
| MC stearic acid | 2.0 % |
| Cetanol | 1.5 % |
| Isopropyl myristate | 10.0 % |
| Paraoxybenzoic acid methyl | 0.2 % |
| Perfume | trace |
| Distilled water | ad. 100 |

| Example 9. Packs | |
|---|---|
| 10-Heptadecenoic acid | 1.0 % |
| Ethanol | 6.0 % |
| Paraoxybenzoic acid methyl | 0.08 % |
| Carboxyvinyl polymer | 1.0 % |
| Calcium carbonate | 0.6 % |
| Titanium oxide | 0.02 % |
| Perfume | trace |
| Distilled water | ad. 100 |

Example 10. Packs

| | |
|---|---|
| 10-Pentadecenoic acid | 1.0 % |
| Ethanol | 6.0 % |
| Paraoxybenzoic acid methyl | 0.08 % |
| Carboxyvinyl polymer | 1.0 % |
| Calcium carbonate | 0.6 % |
| Titanium oxide | 0.02 % |
| Perfume | trace |
| Distilled water | ad. 100 |

| Example 11. Packs | |
|---|---|
| 6-Octadecenol | 1.0 % |
| Ethanol | 6.0 % |
| Paraoxybenzoic acid methyl | 0.08 % |
| Carboxyvinyl polymer | 1.0 % |
| Calcium carbonate | 0.6 % |
| Titanium oxide | 0.02 % |
| Perfume | trace |
| Distilled water | ad. 100 |

| Example 12. Packs | |
|---|---|
| 7,10,13-Heptadecatrienol | 1.0 % |
| Ethanol | 6.0 % |
| Paraoxybenzoic acid methyl | 0.08 % |
| Carboxyvinyl polymer | 1.0 % |
| Calcium carbonate | 0.6 % |
| Titanium oxide | 0.02 % |
| Perfume | trace |
| Distilled water | ad. 100 |

Example 13. Lotions

| | |
|---|---|
| Solution of 6,9-hexadecadienoic acid in liposome (containing 1% 9-hexadecenoic acid) | 0.5 % |
| Paraoxybenzoic acid | 0.08 % |
| Citric acid | 0.3 % |
| Perfume | trace |
| Distilled water | ad. 100 |

| Example 14. Lotions | |
|---|---|
| solution of 6,9-hexadecadienoic acid in liposome (containing 1% 9-hexadecenoic acid) | 5.0 % |
| Paraoxybenzoic acid | 0.08 % |
| Citric acid | 0.3 % |
| Perfume | trace |
| Distilled water | ad. 100 |

| Example 15. Lotions | |
|---|---|
| 11,14-Eicosadienol | 5.0 % |
| Paraoxybenzoic acid | 0.08 % |
| Citric acid | 0.3 % |
| Perfume | trace |
| Distilled water | ad. 100 |

| Example 16. Lotions | |
|---|---|
| 6-Decenol | 0.5 % |
| Paraoxybenzoic acid methyl | 0.08 % |
| Citric acid | 0.3 % |
| Perfume | trace |
| Distilled water | ad. 100 |

*The percentage (%) in these examples are described in % by weight.

The melanin-production inhibiting activity of unsaturated straight-chain fatty acids, unsaturated straight-chain aliphatic alcohols and their derivatives as effective ingredients of the invention is identified in the following experimental example.

Experimental Example: Melanin-production test of B16 cells

B16 cells ($4 \times 10^4$) derived from mouse melanoma are suspended in Eagle MBM medium containing bovine fetal serum (10 ml) for culture in the presence of 5% $CO_2$ at 37°C in 25-$cm^3$ Rou bottle. At day 0 and 3, the medium is replaced for a medium containing each of the above substances of 80 µM/l or 60 µM/l to continue the culture for 5 days. The cells washed with phosphate buffer (pH 7.2) containing 0.8 W/V NaCl, were separated using the solution containing trypsin and EDTA, and filtered to recover the cells. The cells recovered on the filter were measured of their reflection luminous intensity (degree of darkness) at 500 nm with a densitometer.

The results are shown in Table 1.

Table 1

| Sample | Evaluated concentration µM | Whitening degree | Melanin levels µg/$10^6$ cells | Number of cells × $10^4$ cells |
|---|---|---|---|---|
| 2-Butenoic acid | 80 | + | 4.6 | 2.7 |
| 6-Octenoic acid | 80 | + | 4.7 | 2.6 |
| 6-Decenoic acid | 80 | + | 4.8 | 2.6 |
| 7-Undecenoic acid | 80 | + | 4.7 | 2.7 |
| 9-Dodecenoic acid | 80 | + | 4.6 | 2.7 |
| 6,9-Dodecadienoic acid | 80 | + | 4.4 | 2.5 |
| 10-Tridecenoic acid | 80 | + | 3.0 | 2.7 |
| 7,10-Tridecadienoic acid | 80 | + | 3.2 | 2.4 |
| 9-Tetradecenoic acid | 80 | ++ | 0.3 | 2.5 |
| 6,9-Tetradecadienoic acid | 80 | ++ | 0.3 | 2.5 |
| 7,10-Pentadecadienoic acid | 80 | +++ | 0.1 | 2.6 |
| 10-Heptadecenoic acid | 80 | ++ | 0.5 | 2.6 |
| 6,9-Hexadecadienoic acid | 80 | +++ | 0.1 | 2.4 |
| 6,9,12-Hexadecatrienoic acid | 80 | +++ | 0.1 | 2.5 |
| 10-Pentadecenoic acid | 80 | +++ | 0.1 | 2.6 |

| Sample | Evaluated concentration µM | Whitening degree | Melanin levels µg/$10^6$ cells | Number of cells × $10^4$ cells |
|---|---|---|---|---|
| 9-Hexadecenoic acid | 80 | +++ | 0.1 | 3.2 |
| 1-Monooctenoin | 80 | + | 4.5 | 2.6 |
| 1-Monododecenoin | 80 | + | 4.2 | 2.7 |
| 1-Monomyristolein | 80 | ++ | 2.2 | 2.5 |
| 1-Monopentadecenoin | 80 | +++ | 0.1 | 2.4 |
| 1-Monopalmitolein | 80 | +++ | 0.1 | 2.2 |
| 1-Monoheptadecenoin | 80 | ++ | 0.4 | 2.0 |
| 2-Monohexenoin | 80 | + | 4.3 | 2.4 |
| 2-Monoundecenoin | 80 | + | 3.9 | 2.5 |
| 2-Monopentadecenoin | 80 | +++ | 0.1 | 2.2 |
| 2-Monopalmitolein | 80 | +++ | 0.1 | 2.1 |
| 2-Monoheptadecenoin | 80 | ++ | 0.3 | 2.2 |
| 1,2-Didodecenoin | 80 | + | 4.0 | 2.4 |
| 1,2-Dimyristolein | 80 | ++ | 2.8 | 2.5 |
| 1,2-Dipentadecenoin | 80 | +++ | 0.1 | 2.5 |
| 1,2-Dipalmitolein | 80 | +++ | 0.1 | 2.2 |
| 1,3-Dimyristoelin | 80 | ++ | 1.2 | 2.0 |
| 1,3-Dipentadecenoin | 80 | +++ | 0.2 | 2.1 |
| 1,3-Dipalmitolein | 80 | +++ | 0.1 | 2.1 |
| 1,3-Heptadecenoin | 80 | ++ | 1.2 | 2.1 |
| Trimyristolein | 80 | ++ | 1.5 | 2.5 |
| Tripentadecenoin | 80 | +++ | 0.2 | 2.2 |

| Sample | Evaluated concentration μM | Whitening degree | Melanin levels μg/$10^6$ cells | Number of cells × $10^4$ cells |
|---|---|---|---|---|
| Tripalmitolein | 80 | +++ | 0.1 | 2.1 |
| Triheptadecenoin | 80 | ++ | 1.0 | 2.7 |
| Myristoleic acid methyl | 80 | ++ | 1.2 | 2.6 |
| 10-Pentadecenoic acid methyl | 80 | +++ | 0.1 | 2.7 |
| Palmitoleic acid methyl | 80 | +++ | 0.1 | 2.6 |
| 10-Heptadecenoic acid methyl | 80 | ++ | 2.0 | 2.5 |
| L-α-Lecithin dipalmitoleyl | 80 | + | 3.9 | 2.8 |
| L-α-Lecithin dipalmitoelaidoyl | 80 | + | 3.8 | 2.7 |

| Sample | Evaluated concentration µM | Whitening degree | Melanin levels µg/$10^6$ cells | Number of cells × $10^4$ cells |
|---|---|---|---|---|
| 3-Hexenol | 80 | + | 4.2 | 2.4 |
| 6-Decenol | 80 | + | 4.3 | 2.3 |
| 6-Tetradecenol | 80 | ++ | 0.8 | 2.0 |
| 10-Pentadecenol | 80 | +++ | 0.1 | 2.2 |
| 9-Hexadecenol | 80 | +++ | 0.1 | 2.1 |
| 10-Heptadecenol | 80 | ++ | 0.5 | 2.3 |
| 3,6-Octadienol | 80 | + | 4.5 | 2.4 |
| 6,9-Undecadienol | 80 | + | 4.2 | 2.3 |
| 6,9-Tetradecadienol | 80 | ++ | 3.5 | 2.2 |
| 7,10-Pentadecadienol | 80 | +++ | 0.2 | 2.1 |
| 6,9-Hexadecadienol | 80 | +++ | 0.1 | 2.0 |
| 6,9,12-Hexadecatrienol | 80 | +++ | 0.1 | 2.2 |
| 7,10-Heptadecadienol | 80 | +++ | 0.1 | 2.3 |
| 7,10,13-Heptadecatrienol | 80 | +++ | 0.1 | 2.4 |
| Myristoleyl acetate | 80 | + | 4.0 | 2.6 |
| 10-Pentadecenyl acetate | 80 | +++ | 0.1 | 2.2 |
| 9-Hexadecenyl acetate | 80 | +++ | 0.1 | 2.4 |
| 10-Heptadecenyl acetate | 80 | ++ | 2.1 | 2.7 |
| Myristoleylmethane sulfonate | 80 | + | 3.7 | 2.8 |

| Sample | Evaluated concentration µM | Whitening degree | Melanin levels µg/$10^6$ cells | Number of cells $\times 10^4$ cells |
|---|---|---|---|---|
| Palmitoleylmethane sulfonate | 80 | +++ | 0.1 | 2.5 |

| Sample | Evaluated concentration μM | Whitening degree | Melanin levels μg/$10^6$ cells | Number of cells × $10^6$ cells |
|---|---|---|---|---|
| 6-Octadecenoic acid | 60 | ++ | 2.0 | 2.5 |
| 11-Octadecenoic acid | 60 | ++ | 2.4 | 2.9 |
| 11,14-Eicosadienoic acid | 60 | + | 3.0 | 2.4 |
| 8,11,14-Eicosatrienoic acid | 60 | + | 3.5 | 2.2 |
| 11,14,17-Eicosatrienoic acid | 60 | + | 3.2 | 2.1 |
| 5,8,11,14-Eicosatetraenoic acid | 60 | + | 4.0 | 2.4 |
| 5,8,11,14,17-Eicosapentaenoic acid | 60 | + | 3.8 | 2.5 |
| 1-Mono-6-Octadecenoic acid | 60 | ++ | 1.9 | 3.1 |
| 1-Mono-11-Octadecenoic acid | 60 | ++ | 2.1 | 2.7 |
| 1-Mono-11,14-Eicosadienoin | 60 | + | 4.3 | 3.1 |
| 1-Mono-8,11,14-Eicosatrienoin | 60 | + | 3.9 | 3.2 |
| 1-Mono-11,14,17-Eicosatrienoin | 60 | + | 3.8 | 3.1 |
| 1-Mono-8,11,14,17-Eicosatetraenoin | 60 | + | 3.8 | 3.3 |

| Sample | Evaluated concentration µM | Whitening degree | Melanin levels µg/$10^6$ cells | Number of cells × $10^6$ cells |
|---|---|---|---|---|
| 1-Mono-5,8,11,14,17-Eicosapentaenoin | 60 | + | 3.0 | 3.0 |
| 2-Mono-6-Octadecenoin | 60 | ++ | 2.1 | 3.1 |
| 2-Mono-11-Octadecenoin | 60 | ++ | 2.4 | 3.3 |
| 2-Mono-11,14-Eicosadienoin | 60 | + | 3.9 | 3.3 |
| 2-Mono-8,11,14-Eicosatrienoin | 60 | + | 3.6 | 3.0 |
| 2-Mono-11,14,17-Eicosatrienoin | 60 | + | 3.7 | 3.1 |
| 2-Mono-8,11,14,17-Eicosatetraenoin | 60 | + | 3.2 | 3.0 |
| 2-Mono-5,8,11,14,17-Eicosapentaenoin | 60 | + | 3.3 | 3.2 |
| 1,2-Di-6-Octadecenoin | 60 | + | 1.8 | 3.0 |
| 1,2-Di-11-Octadecenoin | 60 | ++ | 2.0 | 3.1 |
| 1,2-Di-11,14-Eicosadecenoin | 60 | + | 3.9 | 3.1 |
| 1,2-Di-8,11,14-Eicosatrienoin | 60 | + | 3.5 | 3.2 |
| 1,2-Di-11,14,17-Eicosatrienoin | 60 | + | 3.2 | 3.2 |
| 1,2-Di-8,11,14,17-Eicosatetraenoin | 60 | + | 3.2 | 3.1 |

| Sample | Evaluated concentration μM | Whitening degree | Melanin levels μg/$10^6$ cells | Number of cells × $10^6$ cells |
|---|---|---|---|---|
| 1,2-Di-5,8,11,14,17-Eicosapentaenoin | 60 | + | 3.0 | 3.2 |
| 1,3-Di-6-Octadecenoin | 60 | ++ | 1.5 | 2.9 |
| 1,3-Di-11-Octadecenoin | 60 | ++ | 2.0 | 3.9 |
| 1,3-Di-11,14-Eicosadienoin | 60 | + | 2.8 | 3.0 |
| 1,3-Di-8,11,14-Eicosatrienoin | 60 | + | 3.0 | 3.2 |
| 1,3-Di-11,14,17-Eicosatrienoin | 60 | + | 3.2 | 3.1 |
| 1,3-Di-8,11,14,17-Eicosatetraenoin | 60 | + | 3.5 | 3.2 |
| 1,3-Di-5,8,11,14,17-Eicosapentaenoin | 60 | + | 3.6 | 3.2 |
| Tri-6-Octadecenoin | 60 | ++ | 2.0 | 2.1 |
| Tri-11-Octadecenoin | 60 | ++ | 2.4 | 3.0 |
| Tri-11,14-Eicosadienoin | 60 | + | 3.9 | 3.2 |
| Tri-8,11,14-Eicosatrienoin | 60 | + | 4.1 | 3.2 |
| Tri-11,14,17-Eicosatrienoin | 60 | + | 3.9 | 3.1 |
| Tri-8,11,14,17-Eicosatetraenoin | 60 | + | 3.8 | 3.0 |

| Sample | Evaluated concentration μM | Whitening degree | Melanin levels μg/$10^6$ cells | Number of cells × $10^6$ cells |
|---|---|---|---|---|
| Tri-5,8,11,14,17-Eicosapentaenoin | 60 | + | 3.7 | 3.2 |
| Di-6-Octadecenoyl-L-α-lecithin | 60 | ++ | 2.6 | 3.1 |
| Di-11-Octadecenoyl-L-α-lecithin | 60 | ++ | 2.4 | 3.1 |
| Di-11,14-Eicosadecenoyl-L-α-lecithin | 60 | + | 4.0 | 3.2 |
| Di-8,11,14-Eicosatrienoyl-L-α-lecithin | 60 | + | 3.9 | 3.1 |
| Di-11,14,17-Eicosatrienoyl-L-α-lecithin | 60 | + | 3.8 | 3.1 |
| Di-5,8,11,14-Eicosatetraenoyl-L-α-lecithin | 60 | + | 3.8 | 3.1 |
| Di-8,11,14,17-Eicosatetraenoyl-L-α-lecithin | 60 | + | 3.7 | 3.1 |
| Di-5,8,11,14,17-Eicosapentaenoyl-L-α-lecithin | 60 | + | 3.9 | 3.2 |
| 6-Octadecenoic acid methyl | 60 | ++ | 1.6 | 3.2 |
| 11-Octadecenoic acid methyl | 60 | ++ | 1.8 | 3.0 |

| Sample | Evaluated concentration µM | Whitening degree | Melanin levels µg/$10^6$ cells | Number of cells × $10^6$ cells |
|---|---|---|---|---|
| 11,14-Eicosadienoic acid methyl | 60 | + | 3.8 | 3.1 |
| 8,11,14-Eicosatrienoic acid methyl | 60 | + | 4.1 | 3.2 |
| 11,14,17-Eicosatrienoic acid methyl | 60 | + | 3.9 | 3.1 |
| 5,8,11,14,17-Eicosatetraenoic acid methyl | 60 | + | 3.6 | 3.0 |
| 5,8,11,14,17-Eicosapentaenoic acid methyl | 60 | + | 3.8 | 3.1 |
| 6-Octadecenol | 60 | ++ | 2.5 | 3.0 |
| 11-Octadecenol | 60 | ++ | 2.5 | 2.8 |
| 6-Octadecenyl acetyl ester | 60 | ++ | 3.0 | 2.6 |
| 11-Octadecenyl acetyl ester | 60 | ++ | 2.6 | 2.8 |
| 11,14-Eicosadienyl acetyl ester | 60 | + | 3.8 | 2.7 |
| 11,14,17-Eicosatrienyl acetyl ester | 60 | + | 3.2 | 2.8 |
| 5,8,11,14-Eicosatetraenyl acetyl ester | 60 | + | 3.6 | 2.6 |

| Sample | Evaluated concentration μM | Whitening degree | Melanin levels μg/$10^6$ cells | Number of cells × $10^6$ cells |
|---|---|---|---|---|
| 5,8,11,17-Eicosapentaenyl acetyl ester | 60 | + | 3.3 | 2.5 |
| Control | 0 | - | 6.1 | 4.2 |

Note: - no change, + weakly white, ++ clearly white, +++ nearly white

The external preparation in accordance with the invention is extremely useful, which can be used safely without any disorders such as rough skin and skin irritation and which has effects on fair-skin making and preventing spots and freckle as well.

## Claims

1. External preparation containing, as effective ingredients, unsaturated straight-chain fatty acids or unsaturated straight-chain aliphatic alcohols or the derivatives thereof possessing carbon atoms of 4 to 20 and unsaturated bonds of 1 to 4 at appropriate positions other than the position of carboxyl group or hydroxyl group of alkyl chain and the terminal position opposite thereto.